**Europäisches Patentamt**

**European Patent Office**

**Office européen des brevets**

(11) Veröffentlichungsnummer: **0 498 509 A2**

# EUROPÄISCHE PATENTANMELDUNG

(21) Anmeldenummer: **92200319.9**

(51) Int. Cl.5: **C07C 227/18**

(22) Anmeldetag: **05.02.92**

(30) Priorität: **08.02.91 DE 4103828**

(43) Veröffentlichungstag der Anmeldung:
**12.08.92 Patentblatt 92/33**

(84) Benannte Vertragsstaaten:
**AT BE CH DE DK ES FR GB GR IT LI LU NL PT SE**

(71) Anmelder: **METALLGESELLSCHAFT Aktiengesellschaft**
**Reuterweg 14**
**W-6000 Frankurt am Main(DE)**

(72) Erfinder: **Hänel, Peter, Dr.**
**Iglauer Weg 12**
**W-6368 Bad Vilbel(DE)**
Erfinder: **Helmrich, Harald, Dr.**
**Ben-Gurion-Ring 156**
**W-6000 Frankturt am Main 56(DE)**
Erfinder: **Summer, Harald, Dr.**
**Moosweg 12**
**A-6300 Wörgl(AT)**
Erfinder: **Naderer, Rainer, Dr.**
**Angartherweg 14**
**A-6300 Wörgl(AT)**

(54) **Verfahren zur Herstellung von Aminocarbonsäureestern.**

(57) Es wird ein Verfahren zur Herstellung von Aminocarbonsäureestern aus Aminosäuren oder N-terminalgeschützten Aminosäuren und Alkoholen beschrieben. Die Aminocarbonsäureester werden dadurch hergestellt, daß man die Aminosäuren entweder direkt im Überschuß des zur Veresterung eingesetzten Alkohols löst oder gelöst in einem organischen Lösungsmittel mit Alkohol umsetzt, wobei man das dabei entstehende Reaktionswasser durch Pervaporation an einer mehrschichtigen Membran aus dem Reaktionsgleichgewicht entfernt.

EP 0 498 509 A2

Die Erfindung bezieht sich auf ein Verfahren zur Herstellung von Aminocarbonsäureestern, auch Aminosäureester genannt, durch die Umsetzung von Aminocarbonsäuren, auch Aminosäuren genannt, oder N-terminalgeschützten Aminosäuren mit Alkoholen.

Es sind bereits Verfahren zur Herstellung einer Reihe von Aminosäureestern bekannt. Dabei werden Aminosäuren oder N-terminalgeschützte Aminosäuren mit Akoholen umgesetzt. Als Lösungsmittel dienen, wenn möglich, die zur Veresterung verwendeten Alkohole selbst. Es werden aber auch Lösungsmittel eingesetzt, welche die Reaktion

$$R^1\text{-NH-A-COOH} + R^2\text{-OH} \langle\text{----}\rangle R^1\text{-NH-A-COO-}R^2 + H_2O$$

worin

A der Rest einer natürlich vorkommenden oder künstlich hergestellten Aminosäure ist, wobei eventuell vorhandene funktionelle Gruppen in der Seitenkette des Restes A durch entsprechende Schutzgruppen geschützt sein können,

$R^1$ den Rest einer Schutzgruppe für die Aminogruppe darstellt, z.B. die sogenannte Z-Schutzgruppe ($R^1 = C_6H_5CH_2OCO\text{-}$), die Trifluoracetylgruppe ($R^1 = CF_3CO\text{-}$), die Formylgruppe ($R^1 = CHO\text{-}$) oder die sogenannte $F_mOC$-Gruppe ($R^1 = $ 9-Fluorenylmethyloxycarbonyl) und

$R^2$ den Rest eines aliphatischen oder aromatischen Alkohols darstellt, z.B. den Methyl-, Ethyl-, Propyl-, Cyclohexyl- oder Benzyl-Rest, durch das destillative Entfernen des Wassers in Form eines azeotropen Gemisches aus dem Reaktionsgleichgewicht auf die Seite des Endprodukts verschieben. Allerdings sind diesem Syntheseverfahren dadurch Grenzen gesetzt, daß erstens die Abtrennung des gebildeten Wassers aus den abdestillierten Alkohol-Wasser-Gemischen in der Regel sehr aufwendig ist, daß zweitens die bevorzugt eingesetzten Lösungsmittel Toluen und Xylen nur ihre Siedebereiche als Reaktionstemperatur zulassen, wodurch wegen der relativ hohen Reaktionstemperaturen zahlreiche Nebenreaktionen ablaufen und daß drittens für viele denkbare Reaktionspartner keine geeigneten Lösungsmittel zur Verfügung stehen. Ein weiterer Nachteil ist, daß die Entfernung des im Überschuß vorhandenen Alkohols Probleme verursachen kann (z.B. bei Verwendung von Benzylalkohol, der einen sehr hohen Siedepunkt besitzt).

Der Erfindung liegt daher die Aufgabe zugrunde, ein Verfahren zu schaffen, das es ermöglicht, das bei der Esterbildung entstehende und immer neu gebildete Reaktionswasser ohne großen apparativen Aufwand schnell sowie nahezu quantitativ abzutrennen, und das es weiterhin ermöglicht, andere als die bisher verwendeten Lösungsmittel einzusetzen.

Die der Erfindung zugrundeliegende Aufgabe wird dadurch gelöst, daß man eine Aminocarbonsäure oder eine N-terminalgeschützte Aminocarbonsäure, vorzugsweise eine Z-gruppengeschützte Aminocarbonsäure, entweder gelöst in einem Überschuß des für die Veresterung verwendeten Alkohols oder gelöst in einem anderen organischen Lösungsmittel mit einem Alkohol umsetzt und das entstehende Reaktionswasser kontinuierlich durch Pervaporation an einer mehrschichtigen Membran, die aus einem 50 bis 300 $\mu$m dicken Träger, einer 30 bis 300 $\mu$m dicken, mit Poren versehenen Stützschicht und einer 0,2 bis 10 $\mu$m dicken, porenfreien Trennschicht besteht, aus dem Reaktionsgemisch entfernt.

Überraschenderweise sind neben den zur Veresterung eingesetzten Alkoholen und den bereits bekannten Lösungsmitteln wie Toluen und Xylen auch andere organische Lösungsmittel für die Veresterung von Aminosäuren bzw. N-terminalgeschützte Aminosäuren verwendbar - wie z.B. lineare und zyklische Äther. Durch die Druckbelastbarkeit der verwendeten mehrschichtigen Membran ist in Abhängigkeit vom verwendeten Lösungsmittel die Anwendung der optimalen Reaktionstemperatur durch die Einstellung des entsprechenden Reaktionsdruckes möglich. Damit werden zum einen Nebenreaktionen weitgehend ausgeschlossen, und zum anderen werden eine Reihe von sehr empfindlichen Aminosäuren, die durch ihre schlechte Löslichkeit bzw. ihre geringe Temperaturstabilität bisher nicht umgesetzt werden konnten, der Veresterung zugänglich.

Aus den Druckschriften EP-PS 0210055 und Chemistry Letters, Seiten 2053 bis 2056, 1987 sind zwar Verfahren zur Veresterung von Karbonsäuren bekannt, bei denen das entstehende Reaktionswasser durch Pervaporation an semipermeablen Membranen entfernt wird. Kennzeichnend für alle diese Verfahren ist aber, daß nur die Umsetzung von Karbonsäuren beschrieben wird, die unter Standardbedingungen (20°C, 760 Torr) flüssig und optisch inaktiv sind. Die Pervaporation des gebildeten Reaktionswassers erfolgt in allen Fällen aus der Dampfphase. Überraschenderweise wurde nun gefunden, daß die Abtrennung von Reaktionswasser durch Pervaporation auch bei der Veresterung von Aminosäuren bzw. N-terminalgeschützten Aminosäuren möglich ist, obwohl diese Verbindungen unter Standardbedingungen als Feststoffe vorliegen.

Die Palette der nach dem erfindungsgemäßen Verfahren herstellbaren Aminosäureester läßt sich dadurch vergrößern, daß man als Lösungsmittel einen Äther einsetzt. In vorteilhafter Weise werden zyklische Äther, vorzugsweise Tetrahydrofuran (THF) oder Dioxan, eingesetzt, da diese eine sehr gute Löslichkeit für eine Reihe von N-terminalgeschützten Aminosäuren besitzen. Zur Steigerung der Raum-Zeit-Ausbeute wird der Reaktionsmischung ein Katalysator zugesetzt. Für diesen Zweck hat sich p-Toluensulfonsäure bewährt. Das erfindungsgemäße Verfahren kann besonders erfolgreich durchgeführt werden, wenn man die Reaktionstemperatur durch Variation des Reaktionsdruckes einstellt; es ist also z.B. möglich, durch Erhöhung des Reaktionsdruckes die Siedetemperatur des THF zu erhöhen und damit bei Weiterverwendung dieses guten Lösungsmittels die Reaktionsgeschwindigkeit zu beschleunigen.

Die nach dem erfindungsgemäßen Verfahren verwendete mehrschichtige Membran für die Pervaporation zeichnet sich dadurch aus, daß deren Träger aus einem Polyestergewebe oder -faservlies, die Stützschicht aus Polyacrylnitril oder einem Polysulfon und die Trennschicht aus teilkristallinem Polyvinylalkohol oder Polyvinylalkohol-Polyvinylacetat-Blockcopolymer besteht. Diese mehrschichtige Membran und insbesondere die Trennschicht zeigt überraschenderweise eine hohe Stabilität gegenüber den eingesetzten Reagenzien, ohne daß ihre Selektivität darunter leiden würde. Unter Selektivität ist dabei die Menge an Lösungsmittel oder Alkohol in Gew.-% zu verstehen, die neben dem Wasser durch die Membran permeiert wird.

Die erfindungsgemäße Herstellung der Aminosäureester kann auf zwei Wegen ablaufen. Bei Weg a) erfolgt die Herstellung dadurch, daß man die N-terminalgeschützte Aminosäure im Überschuß des zur Veresterung vorgesehenen Alkohols löst, wenig Katalysator, vorzugsweise p-Toluensulfonsäure, zugibt und die Reaktion bei einer Reaktionstemperatur von 70 bis 100°C und einem Reaktionsdruck von 1 bis 4 bar durchführt, wobei man ein Vakuum von 10 bis 30 mbar an der Rückseite der Membran anlegt. Wenn kein Reaktionswasser mehr permeiert wird, wird die Reaktion beendet und der überschüssige Alkohol in an sich bekannter Weise abgetrennt. Beim Weg b) erfolgt die Herstellung dadurch, daß man die N-terminalgeschützte Aminosäure in einem zyklischen Äther, vorzugsweise THF, löst. Anschließend werden der zur Veresterung vorgesehene Alkohol und der Katalysator, vorzugsweise p-Toluensulfonsäure, zugesetzt. Man führt die Reaktion vorzugsweise bei einer Reaktionstemperatur von 70-100°C und einem Reaktionsdruck von 1-5 bar durch, wobei man das Reaktionswasser durch einen Permeationsdruck von 10-30 mbar an der Rückseite der Membran abführt und auskondensiert. Die Reaktion wird abgebrochen, wenn der Permeatfluß deutlich nachläßt, und anschließend wird die Reaktionsmischung in an sich bekannter Weise, z.B. durch Destillation und Umkristallisation, zum reinen Endprodukt aufgearbeitet.

Die Herstellung von ungeschützten Aminosäureestern erfolgt in der gleichen Weise nach Weg a) oder Weg b); es muß jedoch eine äquimolare Menge an p-Toluensulfonsäure zugegeben werden.

Das erfindungsgemäße Verfahren wird nachfolgend anhand eines Ausführungsbeispiels näher erläutert.

In das Reaktionsgefäß werden 0,1 Mol Z-geschütztes Threonin, gelöst in 2 l Äthanol (reinst), gegeben. Man fügt als Katalysator 0,005 Mol p-Toluensulfonsäure hinzu. Die Reaktion wird bei einer Reaktionstemperatur von ca. 90°C und einem Reaktionsdruck von ca. 3 bar durchgeführt, wobei man an der Rückseite der Membran mit einer Fläche von 2000 cm$^2$ einen Unterdruck von ca. 10 mbar anlegt. Nach ca. 6 Stunden wird die Reaktion beendet, da kein nennenswerter Permeatfluß mehr zu verzeichnen ist. Man erhält nach dem Abdestillieren des überschüssigen Äthanols und nach Umkristallisation in 90%-iger Ausbeute als reines, kristallines Endprodukt den Äthylester des Threonins.

**Patentansprüche**

1. Verfahren zur Herstellung von Aminocarbonsäureestern durch Veresterung von Aminocarbonsäuren oder N-terminalgeschützten Aminocarbonsäuren mit Alkoholen, dadurch gekennzeichnet, daß man eine Aminocarbonsäure oder eine N-terminalgeschützte Aminocarbonsäure, vorzugsweise eine Z-gruppengeschützte Aminocarbonsäure, entweder gelöst in einem Überschuß des für die Veresterung verwendeten Alkohols oder gelöst in einem anderen organischen Lösungsmittel mit einem Alkohol umsetzt und das entstehende Reaktionswasser kontinuierlich durch Pervaporation an einer mehrschichtigen Membran, die aus einem 50 bis 300 $\mu$m dicken Träger, einer 30 bis 300 $\mu$m dicken, mit Poren versehenen Stützschicht und einer 0,2 bis 10 $\mu$m dicken, porenfreien Trennschicht besteht, aus dem Reaktionsgemisch entfernt.

2. Verfahren nach Anspruch 1, dadurch gekennzeichnet, daß man als Lösungsmittel einen Äther einsetzt.

3. Verfahren nach Anspruch 2, dadurch gekennzeichnet, daß man als Lösungsmittel einen zyklischen Äther einsetzt.

4. Verfahren nach Anspruch 3, dadurch gekennzeichnet, daß man als Lösungsmittel Tetrahydrofuran oder Dioxan einsetzt.

5. Verfahren nach einem der Ansprüche 1 bis 4, dadurch gekennzeichnet, daß man einen Katalysator zusetzt.

6. Verfahren nach Anspruch 5, dadurch gekennzeichnet, daß man p-Toluensulfonsäure als Katalysator zusetzt.

7. Verfahren nach einem der Ansprüche 1 bis 6, dadurch gekennzeichnet, daß man die Reaktionstemperatur durch die Variation des Reaktionsdruckes einstellt.

8. Verfahren nach einem der Ansprüche 1 bis 7, dadurch gekennzeichnet, daß man eine mehrschichtige Membran zur Pervaporation verwendet, deren Träger aus einem Polyestergewebe oder -faservlies, deren Stützschicht aus Polyacrylnitril oder einem Polysulfon und deren Trennschicht aus teilkristallinem Polyvinylalkohol oder Polyvinylalkohol-Polvvinylacetat-Blockcopolymer besteht.